# EUROPEAN PATENT SPECIFICATION

(11) **EP 3 831 484 B1**
(45) Date of publication and mention of the grant of the patent: **01.05.2024**
(21) Application number: 20217230.0
(22) Date of filing: 28.03.2017
(51) Int. Cl.: B01L 3/00, B01J 19/00, C12Q 1/6837, C12Q 1/6844, C12Q 1/6874

(54) **MULTI-PLANE MICROARRAYS**
MIKROARRAYS IN MEHREREN EBENEN
MICRO-RÉSEAUX MULTI-PLANS

(30) Priority: 28.03.2016 US 201662313982 P
(43) Date of publication of application: 09.06.2021
(62) Divisional of application: 17776477.6
(73) Proprietor: Illumina, Inc., San Diego, CA 92122 (US)
(72) Inventor: BOWEN, Shane, San Diego, CA 92122 (US); GRAIGE, Michael, San Diego, CA 92122 (US); HONG, Stanley, San Diego, CA 92122 (US); MOON, John, San Diego, CA 92122 (US); SIU, Merek, San Diego, CA 92122 (US)
(74) Representative: Murgitroyd & Company

(56) References cited:
- WO-A1-03/101618
- WO-A1-2010/039147
- US-A1- 2007 134 784
- US-A1- 2009 186 777

## Description

The present disclosure relates to multi plane microarrays.

### BACKGROUND

Various protocols in biological or chemical research involve performing a large number of controlled reactions on solid support surfaces or within predefined reaction chambers. The desired reactions may then be observed or detected and subsequent analysis may help identify or reveal properties of chemicals involved in the reaction. For example, in some multiplex assays, unknown analytes having identifiable labels (e.g., fluorescent labels) may be exposed to thousands of known probes under controlled conditions. Each known probe may be deposited into a corresponding location on a surface. Observing particular chemical reactions that occur between the known probes and the unknown analyte on the surface may help identify or reveal properties of the analyte. Examples of such protocols include known DNA sequencing processes, such as sequencing-by-synthesis (SBS) or known nucleic acid microarray protocols such as RNA expression analysis or genotyping analysis.

In some conventional fluorescent-detection protocols, an optical system is used to direct an excitation light onto fluorescently-labeled analytes and to also detect the fluorescent signals that may emit from the analytes. The resolution of standard imaging techniques is constrained by the number of pixels available in the detection device, among other things. As such, these optical systems can be relatively expensive and require a relatively large bench-top footprint when detecting surfaces having large collections of analytes. For example, nucleic acid arrays used in genotyping, expression, or sequencing analyses can require detection of millions of different sites on the array per square centimeter. Limits in resolution increase cost and decrease accuracy of these analyses.

Thus, there exists a need for higher resolution apparatus and methods, for example, to detect nucleic acid arrays. The present disclosure addresses this need and provides other advantages as well.

WO03101618 describes a substrate for holding an array of experimental sanples; the substrate comprises an array surface having a plurality of wells for holding respective experimental samples of the microarray, wherein the bottom of each well is at one of a plurality of levels, the bottoms of nearest-neighbour wells being at different levels. This helps to reduce signal overlap or interference between samples in nearest-neighbour wells.

WO2010039147 describes an interferometric protein microarray. US2009186777 describes a biochip claiming increased probe density. US2007134784 describes micro-replicated microarrays.

### BRIEF SUMMARY

The invention is defined by the appended claims.

Some embodiments of the present disclosure relate to biological or chemical analysis and more particularly to systems and methods using detection devices for biological or chemical analysis.

In one aspect, the present disclosure provides an array as defined in claim 1. In a second aspect, the present disclosure provides an array as defined in claim 5. The dependent claims set out features of certain embodiments and examples.

### BRIEF DESCRIPTION OF THE DRAWINGS

Fig. 1A shows a plan view of an example array of features wherein all features are at a common elevation of about 200 nm.
Fig. 1B shows a plan view of two example arrays of features including a first array having features at an elevation of about 200 nm and a second array having features at an elevation of about 1000 nm. The two example arrays have the same lattice pattern and pitch but are offset in x and y dimensions.
Fig. 2 shows a cross section of the two example arrays in Fig. 1B, wherein the features are wells having different depths.
Fig. 3 shows a cross section of the two example arrays in Fig. 1B, wherein the features are posts having different heights.
Fig. 4 shows a cross section of the two example arrays in Fig. 1B, wherein a first subset of the features are posts and a second subset of the features are wells.
Fig. 5 shows example use of epi-illumination to excite luminophores in both a first and second array of wells, and use of a waveguide to selectively excite luminophores in one of the arrays of wells.

### DETAILED DESCRIPTION

The present disclosure provides solid supports that can be used for analytical chemistry and biology. In particular embodiments, a solid support will have a surface with two or more arrays of features, each feature presenting an analyte for detection. The features of a first array will occur at a first elevation and the features of a second array will occur at a different elevation that is distinguishable from the first elevation (e.g. by altering depth of focus). This elevation offset provides for more dense packing of analyte features on a surface than would be resolvable if the combination of arrays were at the same elevation on the surface.

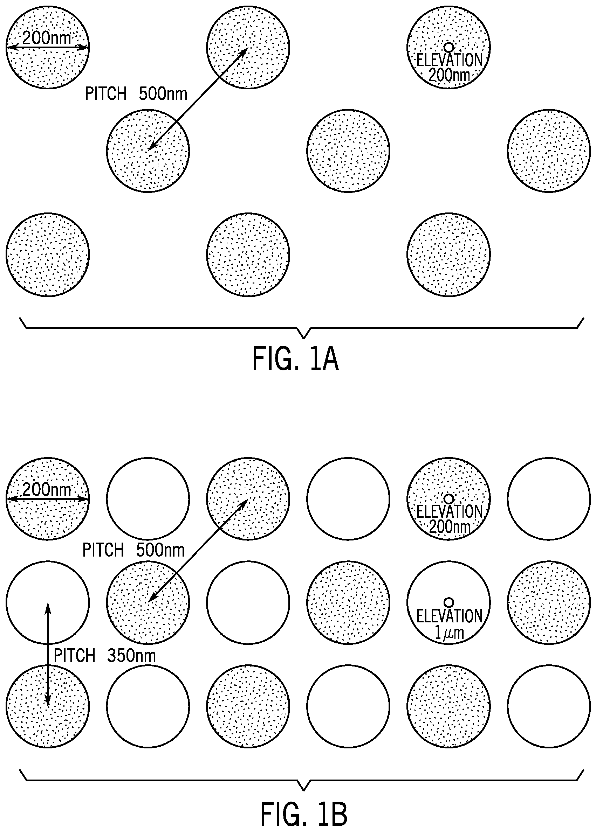

The increase in packing density afforded by the example arrays of the present disclosure is illustrated by Fig. 1A and Fig. 1B. The former figure shows an array having a pitch of about 500 nm. The features of the array are located at a common z-plane (i.e. depth or elevation) and resolvable in the *xy* dimension using commercially available optics. A higher packing of the features in the array of Fig. 1A, would challenge the ability of most optical detectors to distinguish one feature from another. However, in many analytical applications, such as nucleic acid sequencing or other nucleic acid detection techniques, it is desirable to increase the number of analytes in an array to maximize the throughput of analysis in view of time required to fluidically process analytical arrays and to minimize costs given the cost of reagents used to process analytical arrays.

The configuration of the array of Fig. 1B can provide the advantages of increased throughput and decreased costs for fluidic manipulations of the array while avoiding detection limitations that plague other attempts to increase array density. Specifically, as shown in Fig. 1B, the features for two arrays are located at different z-planes (e.g. depth of about 200 nm vs. depth of about 1000 nm) such that features that would otherwise neighbor each other (i.e. if located in the same z-plane) are distinguishable by altering depth of detection. Even more specifically, features in the combined array that are separated by about 350 nm (in the *xy* direction), and that would be difficult to resolve from each other if detected together, can be distinguished by acquiring a first image by focusing an optical detector at a depth of about 200 nm in this example and then acquiring a second image by focusing the optical detector to a depth of about 1 micron in this example.

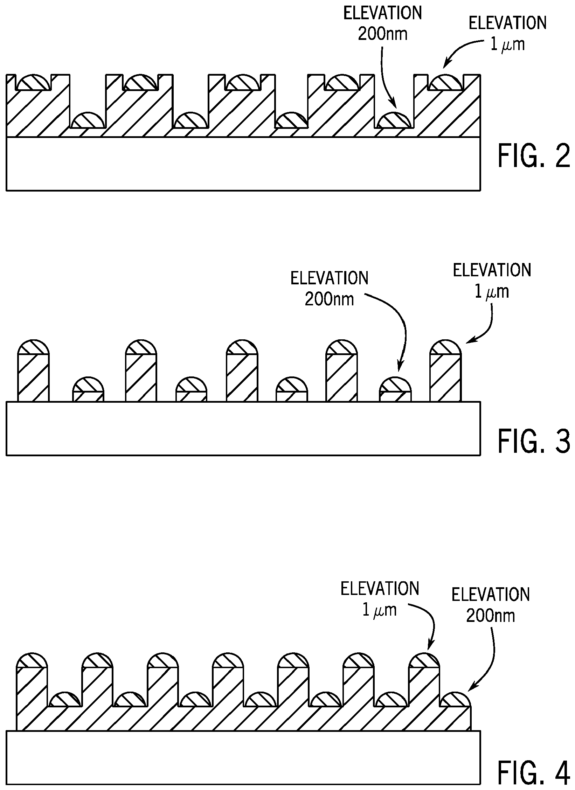

Features that can occur at different *z*-planes include, for example, wells having different depths, as illustrated in the cross section view of an example array presented in Fig. 2. For example, analytes (indicated by semicircles) can be located at the bottom of the wells or at different depths within the wells. In the example configuration of Fig. 2 analytes are attached to the bottoms of a first subset of wells at an elevation of about 200 nm and the second subset of wells positions analytes at an elevation of about 1 micron.

An alternative configuration may be to utilize posts of different heights as shown in the example cross section view of Fig. 3. Analytes (again, indicated by semicircles) can be located at the tops of the posts or at different heights along the length of the posts. In the example configuration of Fig. 3 analytes are attached to the tops of a first subset of posts at an elevation of about 200 nm and the second subset of posts positions analytes at an elevation of about 1 micron. An array can also have a combination of posts and wells, for example, as diagrammed in Fig. 4. It may be noted that in the embodiment of Fig. 3, the features are spaced from one another, while in the embodiment of Fig. 4, the features are adjacent. While in both of these the features may be considered "juxtaposed", in the embodiment of Fig. 4 the features are more densely packed by their adjacent placement with no spaces between them.

Solid supports having two or more arrays in different *z*-planes, can allow for use of lower resolution optics. For example, the features may be larger (and/or further apart) in each of the *z*-planes than discussed above. Arrays of the present disclosure can be made using any of a variety of techniques including, but not limited to, photolithography, nanoimprint lithography or 3D printing.

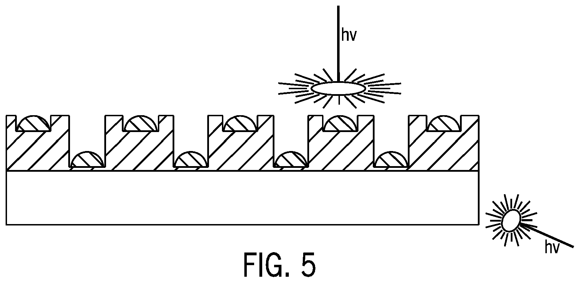

For embodiments that utilize optical detection, such as luminescence, resolution of features can be achieved via both *z*-plane focus of detection and differential *z*-plane excitation. For example, epi-excitation can be combined with total internal reflection (TIR) excitation. Similarly, epi-excitation can be combined with excitation by other waveguides. As illustrated by the diagram in Fig. 5, the waveguide can contact the deeper wells of a first array but not the shallower wells of a second array. Illumination via the waveguide will selectively excite the deeper wells and emission can be collected from only the deep wells. Emission from the shallow wells can be obtained by a subtractive method. Specifically, all wells can be excited by epi-illumination, and the emission from the shallow wells can be identified by subtracting out the emission that was obtained from waveguide illumination of the deeper wells.

Terms used herein will be understood to take on their ordinary meaning in the relevant art unless specified otherwise. Several terms used herein and their meanings are set forth below.

As used herein, the term "amplicon," when used in reference to a nucleic acid, means the product of copying the nucleic acid, wherein the product has a nucleotide sequence that is the same as or complementary to at least a portion of the nucleotide sequence of the nucleic acid. An amplicon can be produced by any of a variety of amplification methods that use the nucleic acid, or an amplicon thereof, as a template including, for example, polymerase extension, polymerase chain reaction (PCR), rolling circle amplification (RCA), multiple displacement amplification (MDA), ligation extension, or ligation chain reaction. An amplicon can be a nucleic acid molecule having a single copy of a particular nucleotide sequence (e.g. a PCR product) or multiple copies of the nucleotide sequence (e.g. a concatameric product of RCA). A first amplicon of a target nucleic acid may be a complementary copy. Subsequent amplicons are copies that are created, after generation of the first amplicon, from the target nucleic acid or from an amplicon. A subsequent amplicon can have a sequence that is substantially complementary to the target nucleic acid or substantially identical to the target nucleic acid.

As used herein the term "analyte" is intended to include any of a variety of analytes that are to be detected, characterized, modified, synthesized, or the like. Example analytes include, but are not limited to, nucleic acids (e.g. DNA, RNA or analogs thereof), proteins, polysaccharides, cells, antibodies, epitopes, receptors, ligands, enzymes (e.g. kinases, phosphatases or polymerases), small molecule drug candidates, or the like. An array can include multiple different species from a library of analytes. For example, the species can be different antibodies from an antibody library, nucleic acids having different sequences from a library of nucleic acids, proteins having different structure and/or function from a library of proteins, drug candidates from a combinatorial library of small molecules etc.

As used herein, the term "attached" refers to the state of two things being joined, fastened, adhered, connected or bound to each other. For example, an analyte, such as a nucleic acid, can be attached to a material, such as a gel or solid support, by a covalent or non-covalent bond. A covalent bond is characterized by the sharing of pairs of electrons between atoms. A non-covalent bond is a chemical bond that does not involve the sharing of pairs of electrons and can include, for example, hydrogen bonds, ionic bonds, van der Waals forces, hydrophilic interactions and hydrophobic interactions

As used herein the term "attachment point" is intended to mean a location on a solid support or gel that is attached to an analyte, capable of being attached to an analyte or intended to be attached to an analyte. The location can accommodate a single analyte or a population of analytes. The location can have an area or dimension(s) as set forth herein for features or contours of an array.

As used herein the term "contour" is intended to mean a localized variation in the shape of a surface. Example contours include, but are not limited to, wells, pits, channels, posts, pillars, and ridges. Contours can occur as any of a variety of depressions in a surface or projections on a surface. All or part of a contour can serve as a feature in an array. For example, a part of a contour that occurs in a particular plane of a solid support can serve as a feature in that particular plane.

As used herein, the term "different", when used in reference to nucleic acids, means that the nucleic acids have nucleotide sequences that are not the same as each other. Two or more nucleic acids can have nucleotide sequences that are different along their entire length. Alternatively, two or more nucleic acids can have nucleotide sequences that are different along a substantial portion of their length. For example, two or more nucleic acids can have target nucleotide sequence portions that are different for the two or more molecules while also having a universal sequence portion that is the same on the two or more molecules. The term can be similarly applied to proteins which are distinguishable as different from each other based on amino acid sequence differences.

As used herein, the term "each," when used in reference to a collection of items, is intended to identify an individual item in the collection but does not necessarily refer to every item in the collection. Exceptions can occur if explicit disclosure or context clearly dictates otherwise.

As used herein, the term "elevation," when used in reference to a feature or contour of a solid support, refers to the height of the feature or contour relative to a reference point or reference level. The reference point or level can be located in or on the solid support, or at a location that is remote from the solid support such as a location where an optical component, such as a lens, resides. Elevation can be determined using a coordinate system. For example, a plane running along an exterior surface of a solid support can be considered as an *xy* plane in a Cartesian Coordinate system, and elevation can be measured along the *z* axis. In another example, the focal plane of an objective lens can be considered as the *xy* plane and elevation can be measured along the axis of the lens.

As used herein, the term "exterior surface" is intended to mean an external part or external layer of a solid support or gel material. The surface can be in contact with another material such as a gas, liquid, gel, polymer, organic polymer, second surface of a similar or different material, metal, or coat. The surface, or regions thereof, can be flat (e.g. taken as an average across an extended surface area). The surface can have surface contours such as wells, pits, channels, ridges, raised regions, pegs, posts or the like.

As used herein, the term "feature" means a location in an array that is configured to attach a particular analyte. The location can be identified in one or more of the *x, y* or *z* dimensions. For example a feature can be all or part of a contour, on a surface, that is present in a particular *z* plane and at a particular *xy* coordinate along the surface. A feature can contain only a single analyte or it can contain a population of several analytes, optionally the several analytes can be the same species. In some embodiments, features are present on a solid support prior to attaching an analyte. In other embodiments the feature is created by attachment of an analyte to the solid support.

As used herein, the term "flow cell" is intended to mean a vessel having a chamber where a reaction can be carried out, an inlet for delivering reagents to the chamber and an outlet for removing reagents from the chamber. In some embodiments the chamber is configured for detection of the reaction that occurs in the chamber (e.g. on a surface that is in fluid contact with the chamber). For example, the chamber can include one or more transparent surfaces allowing optical detection of arrays, optically labeled molecules, or the like in the chamber. Example flow cells include, but are not limited to those used in a nucleic acid sequencing apparatus such as flow cells for the Genome Analyzer^{®}, MiSeq^{®}, NextSeq^{®}, HiSeq^{®}, or NovaSeq^{™} platforms commercialized by Illumina, Inc. (San Diego, CA); or for the SOLiD^{™} or Ion Torrent^{™} sequencing platform commercialized by Life Technologies (Carlsbad, CA). Example flow cells and methods for their manufacture and use are also described, for example, in WO 2014/142841 A1; U.S. Pat. No. 8,241,673, and U.S. Pat. No. 8,951,781, to each of which further reference should be made.

As used herein the term "interleaved," when used in reference to two patterns of features, is intended to mean features of one pattern are interspersed with features of the other pattern, and vice versa, when viewed from a particular perspective. For example, a first pattern of features in a first *xy* plane and a second pattern in a second *xy* plane are interspersed when both *xy* planes are apparent from a particular perspective. In some embodiments, the features of a first pattern can be interspersed with features of a second pattern in a single *xy* plane. Similarly, contours in a first pattern of surface contours can be interleaved with contours of a second pattern of contours on the surface, for example, when portions of the contours are interspersed in a particular *xy* plane.

As used herein the term "lumen," when used in reference to a flow cell, is intended to refer to the cavity within the flow cell. The cavity can be filled with a gas, liquid, gel or combination thereof.

As used herein, the term "luminescent" means emitting cold body radiation. The term is intended to be distinct from incandescence which is radiation emitted from a material as a result of heat. Luminescence results when an energy source displaces an electron of an atom out of its lowest energy ground state into a higher energy excited state; then the electron returns the energy in the form of radiation so it can fall back to its ground state. A particularly useful type of luminescent item is one that emits cold body radiation when energy is provided by excitation radiation. Such items are referred to as "fluorescent" or "photoluminescent". Fluorescence or photoluminescence can be perceived as emission of radiation by an item at a wavelength that is a result of irradiating the item at another wavelength.

As used herein the term "optically coupled" is intended to refer to two things that are aligned or associated to functionally transmit light between the two things. The two things can be in physical contact, but need not be in physical contact. For example, a lens that transmits light to an object through a medium such as air or fluid is considered optically coupled to the object. In some embodiments, light can be modified by one or both things that are optically coupled, for example, by filtering, splitting, polarizing or the like.

As used herein the term "optically detectable" is intended to include, for example, a capability of producing fluorescent, luminescent, scatter, or absorption signals. Optical signals can be detected in the ultraviolet (UV) range (about 200 to about 390 nm), visible (VIS) range (about 391 to about 770 nm), infrared (IR) range (about 0.771 to about 25 microns), or other range of the electromagnetic spectrum. Optical signals can be detected in a way that excludes all or part of one or more of these ranges.

As used herein, the term "pitch," when used in reference to features of an array, is intended to refer to the center-to-center spacing for adjacent features. The term refers to spacing in the *xy* dimension. A pattern of features can be characterized in terms of average pitch. The pattern can be ordered such that the coefficient of variation around the average pitch is small or the pattern can be random in which case the coefficient of variation can be relatively large. Generally speaking, in the context of the disclosure, the average pitch can be, for example, at least about 10 nm, about 0.1 µm, about 0.5 µm, about 1 µm, about 5 µm, about 10 µm, about 100 µm or more. Alternatively or additionally, the average pitch can be, for example, at most about 100 µm, about 10 µm, about 5 µm, about 1 µm, about 0.5 µm, about 0.1 µm or less. Of course, the average pitch for a particular pattern of features can be between one of the lower values and one of the upper values selected from the ranges above.

As used herein, the term "repeating pattern," when used in reference to features, is intended to mean that the relative locations of a subset of features in one region of the object is the same as the relative locations of a subset of features in at least one other region of the object. The repeat occurs in the *x* and *y* dimensions. The one region may be adjacent to that other region in the pattern. The relative locations for features in one region of a repeating pattern are predictable from the relative locations of features in another region of the repeating pattern. The subset used for the measure may include at least 2 features but can include at least, 3, 4, 5, 6, 10 or more features. Alternatively or additionally, the subset used for the measure can include no more than 2, 3, 4, 5, 6, or 10 features. Example repeating patterns include square lattices, rectangular lattices, rhombic lattices, hexagonal lattices and oblique lattices. A repeating pattern can include multiple repetitions of a sub-pattern.

As used herein, reference to "selectively" manipulating (or "selective" manipulation of) a first thing compared to second thing is intended to mean that the manipulation has a greater effect on the first thing compared to the effect on the second thing. The manipulation need not have any effect on the second thing. The manipulation can have an effect on the first thing that is at least about 1%, about 10%, about 50%, about 90%, or about 99% greater than the effect on the second thing. The manipulation can have an effect on the first thing that is at least about 2 fold, about 5 fold, about 10 fold, about 100 fold, about 1×10³ fold, about 1×10⁴ fold or about 1×10⁶ fold higher than the effect on the second thing. The manipulation can include, for example, detecting, exciting, modifying, contacting, treating, changing, cleaving (e.g. of a chemical bond), photo-chemically cleaving (e.g. of a chemical bond), forming (e.g. of a chemical bond), photo-chemically forming (e.g. of a chemical bond), covalently modifying, non-covalently modifying, destroying, photo-ablating, removing, synthesizing, polymerizing, photo-polymerizing, amplifying (e.g. of a nucleic acid), copying (e.g. of a nucleic acid), extending (e.g. of a nucleic acid), ligating (e.g. of a nucleic acid), or other manipulation set forth herein or otherwise known in the art.

As used herein, the term "solid support" refers to a rigid substrate that is insoluble in aqueous liquid. The substrate can be non-porous or porous. The substrate can optionally be capable of taking up a liquid (e.g. due to porosity) but may be sufficiently rigid that the substrate does not swell substantially when taking up the liquid and does not contract substantially when the liquid is removed by drying. A nonporous solid support may be impermeable to liquids or gases. Example solid supports include, but are not limited to, glass and modified or functionalized glass, plastics (e.g. acrylics, polystyrene and copolymers of styrene and other materials, polypropylene, polyethylene, polybutylene, polyurethanes, Teflon^{™}, cyclic olefins, polyimides etc.), nylon, ceramics, resins, Zeonor, silica or silica-based materials including silicon and modified silicon, carbon, metals, inorganic glasses, optical fiber bundles, and polymers. Particularly useful solid supports for some embodiments are components of a flow cell or located within a flow cell apparatus. Example flow cells are set forth in further detail herein.

As used herein the term "waveguide" is intended to mean a structure or material that confines the propagation of electromagnetic radiation to a particular location. For example, a waveguide can guide light to a first subset of features in an array while preventing the light from propagating to a second subset of features in the array. Example waveguides that can be used to couple excitation radiation to features of an array are set forth in US Pat. App. Pub. Nos. 2006/0057729 A1 or 2015/0293021 A1, or US Pat. No. 8,241,573, to each of which further reference should be made.

As used herein the term "window," when used in reference to a flow cell, is intended to mean a wall or barrier of the flow cell through which a fluid or gas cannot pass, but through which radiation of a particular wavelength can transmit.

As used herein, the term "*xy* coordinates" refers to information that specifies the location of a point, line or area along a surface of a solid support. The *xy* coordinates may relate to a plane that is substantially parallel to the surface and/or orthogonal to the *z* dimension along which a detector is focused when viewing the surface. The term can specify, for example, numerical coordinates in a Cartesian system. The coordinates can be provided relative to one or both of the *x* and *y* axes or can be provided relative to another location in the *xy* plane. For example, coordinates of a feature of an object can specify the location of the feature relative to location of another feature of the object.

As used herein, the term "*xy* plane" is intended to mean a 2 dimensional area defined by axes *x* and *y*. One or both of the axes can be straight or curved. For example, a "flat plane" is a 2 dimensional area defined by straight line axes *x* and *y.* When used in reference to a detector and an object observed by the detector, the flat plane can be further specified as being orthogonal to the direction of observation between the detector and object being detected. As used herein, the term "curved plane" is intended to mean a plane that deviates from a flat pane due to an arc in one or both of the *x* or *y* axes. When used in reference to a detector and an object observed by the detector, the curved plane can be further specified as having a tangent that is orthogonal to the direction of observation between the detector and object being detected.

As used herein, the term "*z* coordinate" is intended to mean information that specifies the location of a point, line or area along an axis that is orthogonal to an *xy* plane. In particular embodiments, the *z* axis is orthogonal to an area of an object that is observed by a detector. For example, the direction of focus for an optical system may be specified along the *z* axis.

The embodiments set forth below and recited in the claims can be understood in view of the above definitions.

The present disclosure provides an array, that includes a solid support having a plurality of contours along an exterior surface of the solid support, wherein a first subset of the contours is positioned along the exterior surface of the solid support to form a first pattern of features and a second subset of the contours is positioned along the exterior surface of the solid support to form a second pattern of features, wherein the contours of the first subset are juxtaposed with the contours of the second subset along the exterior surface, whereby the first and second patterns form an interleaved pattern along the exterior surface, wherein the features of the first pattern occur at a first elevation *z₁* and the features of the second pattern occur at a second elevation *z₂*, and wherein the features include attachment points for analytes, whereby the features of the first repeating pattern are configured to attach analytes at a different elevation relative to analytes attached to the features of the second repeating pattern. In some embodiments the pattern of features are repeating patterns of features.

Solid supports that are useful in an apparatus or method of the present disclosure can be two-or three-dimensional and can be a flat surface (e.g., a chip or slide) or can have a curved surface (e.g. a cylinder or drum). Useful materials include glass, quartz, plastic (such as polystyrene (low cross-linked and high cross-linked polystyrene), polycarbonate, polypropylene or poly(methylmethacrylate)), acrylic copolymer, polyamide, silicon, metal (e.g., alkanethiolate-derivatized gold), cellulose, nylon, latex, dextran, gel matrix (e.g., silica gel), polyacrolein, or composites.

As set forth in further detail below, a solid support can be configured to attach to one or more analytes of interest. The attachment can be achieved through direct or indirect bonding to the solid support. The bonding can be by covalent linkage. See Joos et al. (1997) Analytical Biochemistry, 247:96-101; Oroskar et al. (1996) Clin. Chem., 42:1547-1555; and Khandjian (1986) Mol. Bio. Rep., 11:107-11, to each of which further reference should be made. A preferred attachment for nucleic acid analytes, but one that is not limited to nucleic acids, is direct amine bonding, which in the case of nucleic acids can occur between a terminal nucleotide of the nucleic acid to an epoxide integrated on the surface. The bonding also can be through non-covalent linkage. For example, biotin-streptavidin (Taylor et al. (1991) J. Phys. D: Appl. Phys., 24: 1443, to which further reference should be made) and digoxigenin with anti-digoxigenin (Smith et al., Science, 253:1122 (1992), to which further reference should be made) are common tools for anchoring nucleic acids to surfaces.

Attachment of an analyte, such as a nucleic acid, to a surface can be via an intermediate structure such as a bead, particle or gel (e.g. hydrogel). Attachment of nucleic acids to an array via a hydrogel is exemplified by flow cells available commercially from Illumina Inc. (San Diego, CA) or described in US Pat. Nos. 8,241,573 and 8,951,781; and8,759,037, to each of which further reference should be made. Example gels that can be used in the methods and apparatus set forth herein include, but are not limited to, those having a colloidal structure, such as agarose; polymer mesh structure, such as gelatin; or cross-linked polymer structure, such as polyacrylamide, SFA (see, for example, U.S. Pat. App. Pub. No. 2011/0059865 A1, to which further reference should be made) or PAZAM (see, for example, U.S.. Pat. No. 9,012,022, to which further reference should be made). Attachment of nucleic acids to an array via beads is exemplified by BeadChip^{™} arrays available commercially from Illumina Inc. or bead-based arrays set forth in U.S. Patent Nos. 6,266,459; 6,355,431; 6,770,441; 6,859,570; and 7,622,294, to each of which further reference should be made. Such intermediate structures can be present in a well, on a post or at other surface contours set forth herein.

A solid support can include any of a variety of contours. Example contours include, but are not limited to depressions, wells, channels, projections, ridges or posts. Such contours can form features of one or more array. The characteristics of various features are set forth herein. It will be understood that contours can include or impart such characteristics whether or not the contours function as features in an array. Furthermore, a contour can support multiple features such that a contour can have multiple characteristics.

The features of an array can have any of a variety of shapes. For example, when observed in a two dimensional plane, such as on the surface of an array, the features can appear rounded, circular, oval, rectangular, square, symmetric, asymmetric, triangular, polygonal, or the like. The features can be arranged in a regular repeating pattern including, for example, a square lattice, rectangular lattice, rhombic lattice, hexagonal lattice or oblique lattice. A random or non-repeating pattern can also be used in some embodiments. A pattern can be selected to achieve a desired level of packing. For example, round features are optimally packed in a hexagonal arrangement. Of course other packing arrangements can also be used for round features and vice versa.

A repeating pattern can be characterized in terms of the number of features that are present in a subset that forms the smallest repeated unit of the pattern. The subset can include, for example, at least about 2, 3, 4, 5, 6, 10 or more features. Depending upon the size and density of the features the unit can occupy an area of less than about 1 mm², about 500 µm², about 100 µm², about 50 µm², about 10 µm², about 1 µm², about 500 nm², about 100 nm², about 50 nm², about 10 nm² or less. Alternatively or additionally, the unit can occupy an area of greater than about 10 nm², about 50 nm², about 100 nm², about 500 nm², about 1 µm², about 10 µm², about 50 µm², about 100 µm², about 500 µm², about 1 mm², or more. Characteristics of the features in a unit, such as shape, size, pitch and the like, can be selected from those set forth herein more with regard to features in an array or pattern.

The size of a feature on an array (or other object used in a method or system herein) can be selected to suit a particular application. For example, in some embodiments a feature of an array can have a size that accommodates only a single nucleic acid molecule. A surface having a plurality of features in this size range is useful for constructing an array of molecules for detection at single molecule resolution. Features in this size range are also useful for use in arrays having features that each contain a colony of nucleic acid molecules. Thus, the features of an array can each have an area that is no larger than about 1 mm², no larger than about 500 µm², no larger than about 100 µm², no larger than about 10 µm², no larger than about 1 µm², no larger than about 500 nm², or no larger than about 100 nm², no larger than about 10 nm², no larger than about 5 nm², or no larger than about 1 nm². Alternatively or additionally, the features of an array will be no smaller than about 1 mm², no smaller than about 500 µm², no smaller than about 100 µm², no smaller than about 10 µm², no smaller than about 1 µm², no smaller than about 500 nm², no smaller than about 100 nm², no smaller than about 10 nm², no smaller than about 5 nm², or no smaller than about 1 nm². Indeed, a feature can have a size that is in a range between an upper and lower limit selected from those exemplified above. Although several size ranges for features of a surface have been exemplified with respect to nucleic acids and on the scale of nucleic acids, it will be understood that features in these size ranges can be used for applications that do not include nucleic acids. It will be further understood that the size of the features need not necessarily be confined to a scale used for nucleic acid applications.

For an array of features that are present in a particular *z*-plane, the features can be discrete, being separated with spaces between each other. For example, nearest neighbor features in a particular *z*-plane can be separated by edge to edge distance of at most about 100 µm, about 50 µm, about 10 µm, about 5 µm, about 1 µm, about 0.5 µm, about 0.4 µm, about 0.3 µm, about 0.2 µm, about 0.1 µm, or less. Alternatively or additionally, nearest neighbor features in a particular *z*-plane can be separated by an edge to edge distance of at least about 0.1 µm, about 0.2 µm, about 0.3 µm, about 0.4 µm, about 0.5 µm, about 1 µm, about 5 µm, about 10 µm, about 50 µm, about 100 µm or more. These ranges can apply to the average edge to edge spacing for features that are in different *z*-planes as well.

An array can also be characterized with regard to pitch. For example, the size of the features and/or pitch of the features can vary such that arrays can have a desired density. For example, the average feature pitch in a particular z-plane can be at most about 100 µm, about 50 µm, about 10 µm, about 5 µm, about 1 µm, about 0.5 µm, about 0.4 µm, about 0.3 µm, about 0.2 µm, about 0.1 µm or less. Alternatively or additionally, the average feature pitch in a particular *z*-plane can be at least about 0.1 µm, about 0.2 µm, about 0.3 µm, about 0.4 µm, about 0.5 µm, about 1 µm, about 5 µm, about 10 µm, about 50 µm, about 100 µm or more. Similarly, the maximum feature pitch a particular *z*-plane can be at most about 100 µm, about 50 µm, about 10 µm, about 5 µm, about 1 µm, about 0.5 µm about 0.4 µm, about 0.3 µm, about 0.2 µm, about 0.1 µm or less; and/or the minimum feature pitch in a particular *z*-plane can be at least about 0.1 µm, about 0.2 µm, about 0.3 µm, about 0.4 µm, about 0.5 µm, about 1 µm, about 5 µm, about 10 µm, about 50 µm, about 100 µm or more. The above ranges can apply to the average, maximum or minimum pitch between features that are in two or more *z*-planes (e.g. across multiple images in a *z*-stack).

The density of features in an array can also be understood in terms of the number of features present per unit area. For example, the average density of features for an array can be at least about 1 × 10³ features/mm², about 1 × 10⁴ features/mm², about 1 × 10⁵ features/mm², about 1 × 10⁶ features/mm², about 1 × 10⁷ features/mm², about 1 × 10⁸ features/mm², or about 1 × 10⁹ features/mm² or higher. Alternatively or additionally the average density of features for an array can be at most about 1 × 10⁹ features/mm², about 1 × 10⁸ features/mm², about 1 × 10⁷ features/mm², about 1 × 10⁶ features/mm², about 1 × 10⁵ features/mm², about 1 × 10⁴ features/mm², or about 1 × 10³ features/mm² or less. The above ranges can apply to the density of features that are in a single *z*-plane or in multiple *z*-planes(e.g. across multiple images in a *z*-stack).

An array having a regular pattern of features can be ordered with respect to the relative locations of the features but random with respect to one or more other characteristic of each feature. For example, in the case of a nucleic acid array, the nucleic acid features can be ordered with respect to their relative locations but random with respect to one's knowledge of the sequence for the nucleic acid species present at any particular feature. As a more specific example, nucleic acid arrays formed by seeding a repeating pattern of features with template nucleic acids and amplifying the template at each feature to form copies of the template at the feature (e.g. via cluster amplification or bridge amplification) will have a regular pattern of nucleic acid features, as determined by the position of the contours that form the features, but will be random with regard to the distribution of sequences of the nucleic acids across the array. Thus, detection of the presence of nucleic acid material on the array can yield a repeating pattern of features, whereas sequence specific detection can yield non-repeating distribution of signals across the array.

It will be understood that the description herein of patterns, order, randomness and the like pertain not only to features on objects, such as features on arrays, but also to features in images. As such, patterns, order, randomness and the like can be present in any of a variety of formats that are used to store, manipulate or communicate image data including, but not limited to, a computer readable medium or computer component such as a graphical user interface or other output device.

In some embodiments, analytes can be distributed on features such that the analytes are individually resolvable. For example, no more than a single molecule of each analyte may be present at each feature in this embodiment. Alternatively, analytes can be present as colonies or populations such that individual molecules or cells are not necessarily resolved. The colonies or populations can be homogenous with respect to containing only a single species of analyte (albeit in multiple copies). Taking nucleic acids as an example, each feature in an array can include a colony or population of nucleic acids and every nucleic acid in the colony or population can have the same nucleotide sequence (either single stranded or double stranded). Colonies of nucleic acids can also be referred to as "nucleic acid clusters". Nucleic acid colonies can optionally be created by cluster amplification or bridge amplification techniques as set forth in further detail elsewhere herein. Multiple repeats of a target sequence can be present in a single nucleic acid molecule, such as a concatamer created using a rolling circle amplification procedure. Thus, a feature can contain multiple copies of a single species of an analyte. Alternatively, a colony or population of analytes that are at a feature can include two or more different species. For example, one or more features in an array of pads can each contain a mixed colony having two or more different nucleic acid species (i.e. nucleic acid molecules with different sequences). The two or more nucleic acid species in a mixed colony can be present in non-negligible amounts, for example, allowing more than one nucleic acid to be detected in the mixed colony.

A different analyte species may be present at each feature of an array. Thus, different analytes can be present at different features of a first array on a solid support and different analytes can be present at different features of a second array on the solid support. Each array can include at least about 100, about 1 × 10³, about 1 × 10⁴, about 1 × 10⁵, about 1 × 10⁶, about 1 × 10⁹ or more different species of analyte. Accordingly, at least twice as many different analytes can be present on a solid support having two different arrays, at least three fold as many different analytes can be present on a solid support having three different arrays, and so forth.

Any of a variety of analytes can be used in a method or apparatus set forth herein. For example an array can include an attached collection of cells, viruses, nucleic acids, proteins, antibodies, carbohydrates, small molecules (such as drug candidates), biologically active molecules or other analytes of interest.

Useful applications may include those that utilize features having biological molecules such as nucleic acids and polypeptides. Such microarrays may include deoxyribonucleic acid (DNA) or ribonucleic acid (RNA) probes. These are specific for nucleotide sequences present in humans and other organisms. In certain applications, for example, individual DNA or RNA probes can be attached at individual features of an array. A test sample, such as from a known person or organism, can be exposed to the array, such that target nucleic acids (e.g. gene fragments, mRNA, or amplicons thereof) hybridize to complementary probes at respective features in the array. The probes can be labeled in a target specific process (e.g. due to labels present on the target nucleic acids or due to enzymatic labeling of the probes or targets that are present in hybridized form at the features). The array can then be examined by scanning specific frequencies of light over the features to identify which target nucleic acids are present in the sample.

In some embodiments, biological microarrays may be used for genetic sequencing and similar applications. In general, genetic sequencing consists of determining the order of nucleotides in a length of target nucleic acid, such as a fragment of DNA or RNA. Relatively short sequences may be sequenced at each feature, and the resulting sequence information may be used in various bioinformatics methods to logically fit the sequence fragments together so as to reliably determine the sequence of much more extensive lengths of genetic material from which the fragments were derived. Automated, computer-based algorithms for characteristic fragments have been developed, and have been used more recently in genome mapping, identification of genes and their function, and so forth. Microarrays are particularly useful for characterizing genomic content because a large number of variants are present and this supplants the alternative of performing many experiments on individual probes and targets. The microarray is an ideal format for performing such investigations in a practical manner.

Any of a variety of known methods for making analyte arrays (also referred to as "microarrays") or known compositions used for the arrays can be adapted for use in a method or apparatus set forth herein. An array may contain features, each having an individual probe or a population of probes. In the latter case, the population of probes at each feature may be homogenous having a single species of probe. For example, in the case of a nucleic acid array, each feature can have multiple nucleic acid molecules each having a common sequence. However, in some embodiments the populations at each feature of an array can be heterogeneous. Similarly, protein arrays can have features with a single protein or a population of proteins such as having the same amino acid sequence. The probes can be attached to the surface of an array for example, via covalent linkage of the probes to the surface or via non-covalent interaction(s) of the probes with the surface. In some embodiments, probes, such as nucleic acid molecules, can be attached to a surface via a gel layer as described, for example, in U.S. Pat.. No. 9,012,022 and U.S. Pat. App. Pub. No. 2011/0059865 A1, to each of which further reference should be made.

Example arrays include, without limitation, a BeadChip^{®} Array available from Illumina, Inc. (San Diego, CA) or others such as those where probes are attached to beads that are present on a surface (e.g. beads in wells on a surface) such as those described in U.S. Patent Nos. 6,266,459; 6,355,431; 6,770,441; 6,859,570; and 7,622,294, to each of which further reference should be made. Further examples of commercially available microarrays include, for example, an Affymetrix^{®} GeneChip^{®} microarray or other microarray synthesized in accordance with techniques sometimes referred to as VLSIPS^{™} (Very Large Scale Immobilized Polymer Synthesis) technologies. A spotted microarray can also be used in a method or apparatus according to some embodiments of the present disclosure. An example spotted microarray is a CodeLink^{™} Array available from Amersham Biosciences. Another microarray that is useful is one that is manufactured using inkjet printing methods such as SurePrint^{™} Technology available from Agilent Technologies.

Other useful array compositions and manufacturing methods include those that are used in nucleic acid sequencing applications. For example, arrays having amplicons of genomic fragments (often referred to as clusters) are particularly useful such as those described in Bentley et al., Nature 456:53-59 (2008);; WO 91/06678; WO 07/123744; U.S. Pat. Nos. 7,329,492; 7,211,414; 7,315,019; 7,405,281, or 7,057,026; 8,343,746; and 9,574,226, to each of which further reference should be made. Another type of array that is useful for nucleic acid sequencing is an array of particles produced from an emulsion PCR technique. Examples are described in Dressman et al., Proc. Natl. Acad. Sci. USA 100:8817-8822 (2003), WO 05/010145, U.S. Pat... No. 7,323,305 and 7,622,280, to each of which further reference should be made.

Patterned arrays can be used for nucleic acid sequencing or other analytical applications and can be modified for use in the apparatus and methods of the present disclosure. Example patterned arrays, methods for their manufacture and methods for their use are set forth in U.S. Pat... Nos. 8,778,848; 8,778,849; 8,895,249; 9,012,022; and 9,512,422; to each of which further reference should be made. The features of such patterned arrays can be used to capture a single nucleic acid template molecule to seed subsequent formation of a homogenous colony, for example, via bridge amplification. Such patterned arrays are particularly useful for nucleic acid sequencing applications.

The above techniques for making and detecting arrays can be employed when making and using an array having features at multiple z-planes.

Any of a variety of labels or moieties can be present at a feature. Example labels and moieties include, but are not limited to luminophores, fluorophores, chromophores, chemiluminescent species, electrochemical luminescence species, fluorescence quenchers, donors and/or acceptors for fluorescence resonance energy transfer (FRET), nanocrystals and the like. Fluorophores that may be useful include, for example, fluorescent lanthanide complexes, including those of Europium and Terbium, fluorescein, rhodamine, tetramethylrhodamine, eosin, erythrosin, coumarin, methyl-coumarins, pyrene, Malacite green, Cy3, Cy5, stilbene, Lucifer Yellow, Cascade Blue, Texas Red, Alexa dyes, phycoerythin, and others known in the art such as those described in Haugland, Molecular Probes Handbook, (Eugene, OR) 6th Edition; The Synthegen catalog (Houston, TX.), Lakowicz, Principles of Fluorescence Spectroscopy, 2nd Ed., Plenum Press New York (1999), or WO 98/59066, to each of which further reference should be made. Example quenchers include, but are not limited to, DACYL(4-(4'-dimethylaminophenylazo)benzoic acid), Black Hole Quenchers (Biosearch Technologies, Novato, CA), Qxl quenchers (Anaspec, Freemont, CA), Iowa black quenchers, DABCYL, BHQ1, BHQ2, QSY7, QSY9, QSY21, QSY35, BHQO, BHQ1, BHQ2, QXL680, ATTO540Q, ATTO580Q, ATTO612Q, DYQ660, DYQ661 and IR Dye QC-1 quenchers. Chemiluminescent species include, for example, luminal, reagents used for detection in pyrosequencing, aequorin and other species known in the art. Example electrochemical luminescence species include, but are not limited to Ru(bpy₃)²⁺, Bodipy dyes, luminal derivatives, acridine esters and others known in the art.

A label or moiety can be selected to suit a particular application of an apparatus or method set forth herein. For example, the label or moiety can be associated with a target analyte that is present at a feature and detected using a technique set forth herein.

In particular embodiments, target analytes include luminescent moieties and a detector is optically coupled with the array to detect emission from the luminescent moieties. An apparatus that includes the fluorescent moieties can further include an excitation assembly as set forth herein or otherwise known in the art. An apparatus with an excitation assembly is also useful when using fluorescence quenchers, donors and/or acceptors for fluorescence resonance energy transfer (FRET) or nanocrystals.

Two arrays that are on a solid support can be apparently interleaved with each other when viewed in *xy* dimensions independently of elevation differences for the features of the arrays in the *z* dimension. Accordingly, the contours of a first subset of contours on a solid support can intervene nearest-neighbor contours of a second subset of contours on the solid support. Moreover, the contours of the second subset can intervene nearest-neighbor contours of the first subset. In this way, the first and second patterns form an interleaved pattern along the exterior surface of the solid support.

In particular embodiments, a first repeating pattern of contours on a solid support will have the same lattice pattern as a second repeating pattern of contours on the solid support, and the first repeating pattern is offset from the second repeating pattern along the exterior surface of the solid support. Whether or not, the lattice patterns are the same, the area for each of the contours (e.g. the areas that form features) of the first repeating pattern can be the same as the area for each of the contours of the second repeating pattern. Of course the areas for the contours or features can differ within a particular array or between different arrays on a solid support. It will be further understood that a first repeating pattern can optionally have the same pitch as the second repeating pattern.

A first subset of contours on a solid support can include wells or channels having a bottom at elevation *z₁* and a second subset of contours on the solid support can include wells or channels having a bottom at elevation *z₂*. Similarly, a first subset of contours on a solid support can include posts or ridges having a top at elevation *z₁* and a second subset of contours on the solid support can include posts or ridges having a top at elevation *z₂*. As illustrated by these examples, the subsets of contours on a solid support can be the same shape or morphology. Alternatively, different subsets of contours can have different shapes or morphologies. Accordingly, the contours of a first subset can include wells or channels having a bottom at elevation *z₁* and the contours of a second subset can include posts or ridges having a top at elevation *z₂*. Example configurations are shown in Figs. 2 through 4.

The difference in elevations for contours of two different subsets (e.g. in *z₁* and *z₂*) can differ by any amount appropriate or desired to achieve resolution between two different arrays formed by the contours. In some cases nearest neighbor features will be separated by the optical resolution volume element of the detection system being used. For example, *z₁* and *z₂* (or the distance between nearest neighbor features in z) can be at least about 0.2 µm, about 0.5 µm, about 1 µm, about 2 µm, about 5 µm, about 10 µm, about 25 µm or further apart. Alternatively or additionally, the distance can at most about 25 µm, about 10 µm, about 5 µm, about 2 µm, about 1 µm, about 0.5 µm, about 0.1 µm apart or less.

In some embodiments, a first pattern of features will be in a first flat plane along a solid support and a second pattern of features on the solid support will be in a second flat plane that is parallel to the first flat plane. The planes formed by the features need not be flat. For example, a first pattern of features can form a first curved plane (e.g. along the exterior of a cylinder or drum) and a second pattern of features can occur in a second curved plane. The two curved planes can optionally be substantially concentric with respect to each other.

An array of the present disclosure can be contacted with a fluidic sample. Bulk contact can occur whereby the features of the first and second patterns along a solid support are in simultaneous, fluid communication with a fluid. As such, fluidic processing occurs simultaneously for both of the arrays that are on the surface of the solid support. A flow cell is particularly useful for treating an array with fluid reagents. Accordingly, this disclosure provides a flow cell that houses an array set forth herein such that the exterior surface of the solid support is in contact with the lumen of the flow cell. As such, the features of the arrays can be in contact with the lumen of the flow cell, independent of differences in their elevations in the z-dimension. In particular embodiments, two or more patterns of features, although being present at different elevations, will be present on the same side of the lumen of a flow cell. Thus, a single window of a flow cell can have an array with two or more patterns of features, wherein the features are at different elevations.

A flow cell can optionally include fluidic inlet(s) or outlet(s) to allow for introduction or removal of various fluids, for example, fluids containing reagents used in methods for depositing, synthesizing, labeling or detecting analytes. Various Example methods are set forth in further detail elsewhere herein such as nucleic acid amplification, detection and sequencing. A flow cell can further include windows for detection of analytes at the features of an array. In some embodiments, the solid support that contains contours can be transparent to particular wavelengths of light used for optical detection of analytes at the contours. A flow cell can include one or more detection windows. For example, a first window of the flow cell and a second window of the flow cell can be parallel to each other, the first window being separated from the second window by the lumen of the flow cell.

One or both of the windows of a flow cell can function as solid supports upon which arrays of the present disclosure are formed. The arrays can be present on a surface of the window that contacts the lumen of the flow cell. Thus, the present disclosure provides an array, that includes a solid support having a plurality of contours along an exterior surface of the solid support, wherein a first subset of the contours is positioned along the exterior surface of the solid support to form a first pattern of features and a second subset of the contours is positioned along the exterior surface of the solid support to form a second pattern of features, wherein the contours of the first subset are juxtaposed with the contours of the second subset along the exterior surface, whereby the first and second patterns form an interleaved pattern along the exterior surface, wherein the features of the first pattern occur at a first elevation *z₁* and the features of the second pattern occur at a second elevation *z₂*, and wherein the features include attachment points for analytes, whereby the features of the first pattern are configured to attach analytes at a different elevation relative to analytes attached to the features of the second pattern, wherein the solid support functions as a first window of a flow cell and the flow cell includes a second window that is parallel to the first window, the first window being separated from the second window by the lumen of the flow cell, wherein the second window functions as a second exterior surface that includes a third subset of contours positioned along the second exterior surface to form a third pattern of features and a fourth subset of contours along the second exterior surface to form a fourth pattern of features, wherein the contours of the third subset are juxtaposed with the contours of the fourth subset along the second exterior surface, whereby the third and fourth patterns form a second interleaved pattern along the second exterior surface, and wherein the features of the third pattern occur at a third elevation *z₃* and the features of the fourth pattern occur at a fourth elevation *z₄*. Methods for making and detecting arrays located on multiple windows of a flow cell are known in the art, as set forth for example, in U.S. Pat. No. 8,039,817 (to which further reference should be made) and can be readily modified for use with the methods and apparatus set forth herein

The present disclosure further provides a method of detecting a plurality of analytes, including the steps of (a) providing an array including a solid support that has a first pattern of analyte features and a second pattern of analyte features along an exterior surface of the solid support, wherein the analyte features of the first pattern occur at a first elevation *z₁* and the analyte features of the second pattern occur at a second elevation *z₂*, and wherein the first and second patterns form an interleaved pattern along the exterior surface, (b) detecting optical signals at the first elevation *z₁*, whereby individual analyte features of the first pattern are distinguished from each other; (c) detecting optical signals at the second elevation *z₂*, whereby individual analyte features of the second pattern are distinguished from each other, wherein analyte features in the first pattern are distinguished from nearest-neighbor analyte features in the second pattern by selectively detecting features at the first elevation *z₁* compared to features at the second elevation *z₂*. Optionally, the first and/or second pattern of analyte features can be a repeating pattern of analyte features.

A method of the present disclosure can include a step of detecting optical signals from an array, for example, by obtaining an image. Detection apparatus that are capable of high resolution imaging of surfaces are particularly useful. In particular embodiments, the detection apparatus will have sufficient resolution to distinguish features at the densities, pitches and/or feature sizes set forth herein. Particularly useful are detection apparatus capable of obtaining images or image data from surfaces. Example detectors are those that are configured to maintain an object and detector in a static relationship while obtaining an area image. Scanning apparatus can also be used.

Embodiments described herein may utilize a step-and-shoot procedure in which different portions of an array (in an *xy* plane) are individually detected or imaged between (or after) relative movements of the detector and sample. For example, each area of the array can be excited with a laser or other appropriate radiation source and emission can be detected. Examples of step-and-shoot optical components that can be modified in accordance with the present disclosure are set forth in U.S. Pat.. No. 8,951,781, to which further reference should be made. Embodiments described herein may utilize a scanning procedure in which different portions of an array (in an *xy* plane) are detected or imaged during movement between the features and optical components. In some embodiments, the imaging assembly includes a scanning time-delay integration (TDI) system. Furthermore, the imaging sessions may include line-scanning such that a linear focal region of light is scanned (in an *xy* plane) across the array of pads. Some methods of line-scanning that can be modified in accordance with the present disclosure are described, for example, in U.S. Patent Nos. 7,329,860 and 8,039,817, to each of which further reference should be made. Scanning may also include moving a point focal region of light in a raster pattern across an array of features. Whether using step-and-shoot, scanning, static image collection or other configurations, embodiments can be configured for epi-fluorescent imaging or total-internal-reflectance-fluorescence (TIRF) imaging. Example optical components and arrangements that can be modified in this regard are set forth in U.S. Pat. Nos. 7,329,860; 8,241,573; 8,951,781; and 9,193,996 , to each of which further reference should be made.

Certain embodiments include objective lenses having high numerical aperture (NA) values. Example high NA ranges for which embodiments may be particularly useful include NA values of at least about 0.6. For example, the NA may be at least about about 0.65, about 0.7, about 0.75, about 0.8, about 0.85, about 0.9, about 0.95, or higher. Those skilled in the art will appreciate that NA, being dependent upon the index of refraction of the medium in which the lens is working, may be higher including, for example, up to about 1.0 for air, about 1.33 for pure water, or higher for other media such as oils. However, other embodiments may have lower NA values than the examples listed above. Image data obtained by the optical assembly may have a resolution that is between about 0.1 and about 50 microns or, more particularly, between about 0.1 and about 10 microns. Optical assemblies may have a resolution that is sufficient to individually resolve the features or sites that are separated by a distance of less than about 15 µm, about 10 µm, about 5 µm, about 2 µm, about 1 µm, about 0.5 µm, about 0.2 µm or less.

An advantage of using arrays of the present disclosure, in which contours of interleaved arrays display features at different elevations, is that features can be resolved even though the contours to which they are attached are closer together in the apparent *xy* dimensions than would be possible had the features all had the same elevation. As such, nearest-neighbor contours in an interleaved pattern that are distinguished from each other can have a pitch that is less than about 500 nm, about 250 nm, about 100 nm, about 50 nm or less. Alternatively or additionally, the pitch for nearest-neighbor contours of an interleaved pattern can have a pitch that is greater than about 50 nm, about 100 nm, about 250 nm, about 500 nm or more.

Features that are detected and resolved using a method or apparatus herein can each have an area in the *xy* plane that is smaller than about 100 µm², about 10 µm², about 1 µm², about 0.1 µm² or smaller. Alternatively or additionally, features can each have an area in the *xy* plane that is at least about 0.1 µm², about 1 µm², about 10 µm², about 100 µm², or larger.

In general, the NA value of an objective lens is a measure of the breadth of angles for which the objective lens may receive light. The higher the NA value, the more light that may be collected by the objective lens for a given fixed magnification. This is because the collection efficiency and the resolution increase. As a result, multiple features may be distinguished more readily when using objectives lenses with higher NA values. Therefore, in general, a higher NA value for the objective lens may be beneficial for imaging.

A detector that is used in a method set forth herein can be configured to selectively detect features on a solid support in one *z*-plane versus other *z*-planes along the solid support. For example, a detector can be configured for confocal detection or other detection modes that collect signals in a particular z position while rejecting signals at *z* positions above and below the position being detected. A particularly useful detection apparatus that can be used for line scanning in a defined *z*-plane, and optionally using a time delayed integration format, is described in U.S. Pat. App. Pub. No. 2016/019693, to which further reference should be made.

In some embodiments, features that occur in different *z*-planes can be distinguished by deconvolution. For example, a z-stack of images can be compared to one another to determine the z-plane(s) where each feature resides or to eliminate out of focus features. Example image deconvolution techniques are described in Sibarita JB, Deconvolution microscopy. Adv Biochem Eng Biotechnol. 95 (2005) 201-43, or McNally JG et al., Three-dimensional imaging by deconvolution microscopy. Methods 19: 3 (1999) 373-85, to each of which further reference should be made.

The width of the detection area in *z* can be configured for selective detection of features having elevation differences set forth herein. For example, the width in *z* for the focal area (e.g. thickness in *z* for a focal plane, in the case of an *xy* area detector) can be less than about 25 µm, about 10 µm, about 5 µm, about 2 µm, about 1 µm, about 0.5 µm or about 0.1 µm. Alternatively or additionally, the width in z can be at least about 0.1 µm, about 0.5 µm, about 1 µm, about 2 µm, about 10 µm, about 25 µm or more.

It may be desirable to use a detector having a focal plane with a z thickness that is less than the distance between *z₁* and *z₂* where *z₁* is the elevation of features of a first array on a solid support and *z₂* is the elevation of features of a second array on the solid support. Accordingly, analyte features in the first array are distinguished from nearest-neighbor analyte features in the second array by selectively focusing the optical detector to the first elevation *z₁* compared to the second elevation *z₂*. Similarly, analyte features in the second array are distinguished from nearest-neighbor analyte features in the first array by selectively focusing an optical detector to the second elevation *z₂* compared to the first elevation *z₁*.

Distinguishing features at different elevations can be achieved via selective signal acquisition, for example, using detector focusing, confocality or other techniques set forth above. Alternatively or additionally, resolution in *z* can be achieved by selective excitation of luminophores at a first elevation compared to luminophores at a second elevation. For example, analyte features in a first array can be distinguished from nearest-neighbor analyte features in a second array by selectively exciting luminophores at a first elevation *z₁* compared to a second elevation *z₂*. Such selective excitation can be achieved using waveguides that are selectively coupled to features at the first elevation *z₁* compared to features at the second elevation *z₂*. Similarly, analyte features in the second array can be distinguished from nearest-neighbor analyte features in the first array by selectively exciting luminophores at the second elevation *z₂* compared to the first elevation *z₁*.

As exemplified in Fig. 5, differential excitation of arrays can be achieved by alternating application of waveguide illumination and epi-illumination. In the example shown, epi-illumination is used to excite luminophores in both a first and second array of wells. A waveguide is used to selectively excite luminophores in the second array of wells (the features of the first array are not optically coupled to the waveguide). Thus, the features in the second array are distinguished from the features of the first array by selective excitation via the waveguide and the features in the first array can be selectively detected by image processing to subtract the features of the second array (the locations of which are identified from the waveguide excited image).

A method of the present disclosure can further include a step of providing data from a target image to a computer. Various processes and steps of the methods set forth herein can be carried out using a computer. The computer can include a processor that is part of a detection device, networked with a detection device used to obtain the data that is processed by the computer or separate from the detection device. In some embodiments, information (e.g., image data) may be transmitted between components of a system disclosed herein directly or via a computer network. A Local Area Network (LAN) or Wide Area Network (WAN) may be a corporate computing network, including access to the Internet, to which computers and computing devices comprising the system are connected. In one embodiment, the LAN conforms to the Transmission Control Protocol/Internet Protocol (TCP/IP) industry standard. In some instances, the information (e.g., image data) is input to a system disclosed herein via an input device (e.g. disk drive, compact disk player, USB port etc.). In some instances, the information is received by loading the information, e.g., from a storage device such as a disk or flash drive.

A processor that is used to run an algorithm or other process set forth herein may comprise a microprocessor. The microprocessor may be any conventional general purpose single- or multi-chip microprocessor such as a Pentium^{™} processor made by Intel Corporation. A particularly useful computer can utilize an Intel Ivybridge dual-12 core processor, LSI raid controller, having 128GB of RAM, and 2TB solid state disk drive. In addition, the processor may comprise any conventional special purpose processor such as a digital signal processor or a graphics processor. The processor may have conventional address lines, conventional data lines, and one or more conventional control lines.

The embodiments disclosed herein may be implemented as a method, apparatus, system or article of manufacture using standard programming or engineering techniques to produce software, firmware, hardware, or any combination thereof. The term "article of manufacture" in this context refers to code or logic implemented in hardware or computer readable media such as optical storage devices, and volatile or non-volatile memory devices. Such hardware may include, but is not limited to, field programmable gate arrays (FPGAs), application-specific integrated circuits (ASICs), complex programmable logic devices (CPLDs), programmable logic arrays (PLAs), microprocessors, or other similar processing devices. In particular embodiments, information or algorithms set forth herein are present in non-transient storage media. In particular embodiments, a computer implemented method set forth herein can occur in real time while multiple images of an object are being obtained. Such real time analysis is particularly useful for nucleic acid sequencing applications wherein an array of nucleic acids is subjected to repeated cycles of fluidic and detection steps. Analysis of the sequencing data can often be computationally intensive such that it can be beneficial to perform the methods set forth herein in real time or in the background while other data acquisition or analysis algorithms are in process. Example real time analysis methods that can be used with the present methods are those used for the MiSeq and HiSeq sequencing devices commercially available from Illumina, Inc. (San Diego, CA) and/or described in U.S. Pat. No. 8,965,076, to which further reference should be made.

A method of the present disclosure can include a step of capturing analytes, such as nucleic acids to form analyte features on a solid support surface. In some embodiments nucleic acids (or other analytes) are in a fluid that is contacted with a solid support. The nucleic acids are captured from the fluid such that they randomly end up at particular features on the surface. As such, the features to which the fragments attach can be random with regard to predictability or knowledge of where any specific nucleic acid species will attach (i.e. what nucleic acid sequence will be present at a particular location), whether or not the spatial pattern of the features is random.

Analytes that are in a fluid can be transported randomly to features on a surface via passive diffusion or active transport. Example conditions and techniques that can be used for such transport are known in the art or exemplified herein in the context of transporting modified nucleic acids to a surface.

A method of the present disclosure can further include a step of amplifying nucleic acids at features to produce amplicons. The amplicons can be detected, for example, via a nucleic acid sequencing technique as set forth below. In particular embodiments, nucleic acids can be amplified using at least one primer that is attached to the surface. A primer that is used for amplification can, at least in some configurations, hybridize to a priming site on a nucleic acid. The primer can be extended to produce the amplicons that are attached to the surface. Solid-phase extension methods can be used as set forth in further detail below.

A method of the present disclosure can include a step of amplifying portions of a target nucleic acid, modified nucleic acid, or fragments thereof. Any suitable amplification methodology known in the art can be used. In some embodiments, nucleic acids are amplified on a feature that is present on a solid support. For example, in some embodiments, the nucleic acids are amplified using bridge amplification methodologies as exemplified by the disclosures of U.S. Pat. Nos. 5,641,658; 7,115,400; 7,741,463; 7,790,418; U.S. Patent Publ. Nos. 2002/0055100; 2004/0002090; and 2008/0009420, to each of which further reference should be made. Bridge amplification methods allow amplification products to be immobilized on a solid support in order to form arrays comprised of clusters (or "colonies") of immobilized nucleic acid molecules. Each cluster or colony on such an array is formed from a plurality of identical immobilized polynucleotide strands and a plurality of identical immobilized complementary polynucleotide strands. The arrays so-formed can be referred to herein as "clustered arrays". The products of solid-phase amplification reactions are so-called "bridged" structures when formed by annealed pairs of immobilized polynucleotide strands and immobilized complementary strands, both strands being immobilized on the solid support at the 5' end, preferably via a covalent attachment. Bridge amplification methodologies are examples of methods wherein an immobilized nucleic acid template is used to produce immobilized amplicons. Other suitable methodologies can also be used to produce immobilized amplicons from immobilized nucleic acids produced according to the methods provided herein. For example one or more clusters or colonies can be formed via solid-phase polymerase chain reaction (PCR), solid-phase multiple displacement amplification (MDA), solid-phase rolling circle amplification (RCA) etc. whether one or both primers of each pair of amplification primers are immobilized.

In some embodiments, target nucleic acids, modified nucleic acids, or fragments thereof are amplified in solution. For example, in some embodiments, amplification primers are hybridized to priming sites of inserts in solution. In other embodiments, amplification primers are hybridized to the inserts when modified nucleic acids or fragments thereof are attached to a solid support. Nucleic acids can be amplified in solution prior to being attached to features. The attached amplicons can optionally be amplified a second time using solid phase amplification methods set forth herein.

It will be appreciated that any of the amplification methodologies described herein or known in the art can be utilized with universal or target-specific primers to amplify immobilized DNA fragments. Suitable methods for amplification include, but are not limited to, the polymerase chain reaction (PCR), strand displacement amplification (SDA), transcription mediated amplification (TMA) and nucleic acid sequence based amplification (NASBA), for example, as described in U.S. Patent No. 8,003,354, to which further reference should be made. The above amplification methods can be employed to amplify one or more nucleic acids of interest. For example, PCR, multiplex PCR, SDA, TMA, NASBA and the like can be utilized to amplify immobilized nucleic acid fragments. In some embodiments, primers directed specifically to the nucleic acid of interest are included in the amplification reaction.

Other suitable methods for amplification of nucleic acids can include oligonucleotide extension and ligation, rolling circle amplification (RCA) (Lizardi et al., Nat. Genet. 19:225-232 (1998), to which further reference should be made) and oligonucleotide ligation assay (OLA) (See U.S. Pat. Nos. 7,582,420, 5,185,243, 5,679,524 and 5,573,907; and in EP 0 320 308 B1; EP 0 336 731 B1; EP 0 439 182 B1; WO 90/01069; WO 89/12696; and WO 89/09835, to each of which further reference should be made). It will be appreciated that these amplification methodologies can be designed to amplify immobilized nucleic acids. For example, in some embodiments, the amplification method can include ligation probe amplification or oligonucleotide ligation assay (OLA) reactions that contain primers directed specifically to the nucleic acid of interest. In some embodiments, the amplification method can include a primer extension-ligation reaction that contains primers directed specifically to the nucleic acid of interest. As a non-limiting example of primer extension and ligation primers that can be specifically designed to amplify a nucleic acid of interest, the amplification can include primers used for the GoldenGate^{®} assay (Illumina, Inc., San Diego, CA) as exemplified by U.S. Pat. Nos. 7,582,420 and 7,611,869, to each of which further reference should be made.

An isothermal amplification technique can be used in a method of the present disclosure. Example isothermal amplification methods include, but are not limited to, Multiple Displacement Amplification (MDA) as exemplified by, for example, Dean et al., Proc. Natl. Acad. Sci. USA 99:5261-66 (2002) or isothermal strand displacement nucleic acid amplification as exemplified by, for example U.S. Pat. No. 6,214,587, to each of whichfurther reference should be made. Other non-PCR-based methods that can be used in the present disclosure include, for example, strand displacement amplification (SDA) which is described in, for example Walker et al., Molecular Methods for Virus Detection, Academic Press, Inc., 1995; U.S. Pat. Nos. 5,455,166, and 5,130,238, and Walker et al., Nucl. Acids Res. 20:1691-96 (1992) or hyperbranched strand displacement amplification which is described in, for example Lage et al., Genome Research 13:294-307 (2003), to each of which further reference should be made. Additional description of amplification reactions, conditions and components are set forth in U.S. Patent No. 7,670,810, to which further reference should be made. Other useful isothermal amplification techniques include recombinase-facilitated amplification techniques such as those sold commercially as TwistAmp^{™} kits by TwistDx (Cambridge, UK). Useful components of recombinase-facilitated amplification reagent and reaction conditions are set forth in U.S. Pat. Nos. 5,223,414 and 7,399,590, to each of which further reference should be made. Helicase dependent amplification can also be used, for example, as described in Xu et al. EMBO Rep 5:795-800 (2004), to which further reference should be made.

The methods described herein can include a step of sequencing nucleic acids on arrays. One example is sequencing-by-synthesis (SBS). In SBS, extension of a nucleic acid primer along a nucleic acid template is monitored to determine the sequence of nucleotides in the template. The primer can hybridize to a priming site that is present in an insert as set forth above. The underlying chemical process can be polymerization (e.g. as catalyzed by a polymerase enzyme). In a particular polymerase-based SBS embodiment, fluorescently labeled nucleotides are added to a primer (thereby extending the primer) in a template dependent fashion such that detection of the order and type of nucleotides added to the primer can be used to determine the sequence of the template. A plurality of different nucleic acid fragments that have been attached at different locations of an array using steps set forth herein can be subjected to an SBS technique under conditions where events occurring for different templates can be distinguished due to their location in the array.

Flow cells provide a convenient format for housing an array of nucleic acids that is produced by the methods of the present disclosure and that is subjected to an SBS or other detection technique that involves repeated delivery of reagents in cycles. For example, to initiate a first SBS cycle, one or more labeled nucleotides, DNA polymerase, etc., can be flowed into/through a flow cell that houses an array of nucleic acids. Those features of an array where primer extension (e.g. via hybridization of the primer to a priming site located on an insert attached to a nucleic acid) causes a labeled nucleotide to be incorporated can be detected. Optionally, the nucleotides can further include a reversible termination property that terminates further primer extension once a nucleotide has been added to a primer. For example, a nucleotide analog having a reversible terminator moiety can be added to a primer such that subsequent extension cannot occur until a deblocking agent is delivered to remove the moiety. Thus, for embodiments that use reversible termination, a deblocking reagent can be delivered to the flow cell (before or after detection occurs). Washes can be carried out between the various delivery steps. The cycle can then be repeated n times to extend the primer by n nucleotides, thereby detecting a sequence of length n. Example SBS procedures, fluidic systems and detection platforms that can be readily adapted for use with an array produced by the methods of the present disclosure are described, for example, in Bentley et al., Nature 456:53-59 (2008), U.S. Pat. Nos. 7,057,026; 7,329,492; 7,211,414; 7,315,019; 7,405,281; 8,343,746; and in WO 04/018497; WO 91/06678; WO 07/123744; to each of which further reference should be made.

Other sequencing procedures that use cyclic reactions can be used, such as pyrosequencing. Pyrosequencing detects the release of inorganic pyrophosphate (PPi) as particular nucleotides are incorporated into a nascent nucleic acid strand (Ronaghi, et al., Analytical Biochemistry 242(1), 84-9 (1996); Ronaghi, Genome Res. 11(1), 3-11 (2001); Ronaghi et al. Science 281(5375), 363 (1998); U.S. Pat. Nos. 6,210,891; 6,258,568; and. 6,274,320, to each of which further reference should be made). In pyrosequencing, released PPi can be detected by being converted to adenosine triphosphate (ATP) by ATP sulfurylase, and the level of ATP generated can be detected via luciferase-produced photons. Thus, the sequencing reaction can be monitored via a luminescence detection system. Excitation radiation sources used for fluorescence based detection systems are not necessary for pyrosequencing procedures. Useful fluidic systems, detectors and procedures that can be used for application of pyrosequencing to methods of the present disclosure are described, for example, in U.S. Pat. Nos. 7,244,559; 7,595,883; and 9,096,899 and U.S. Pat. Pub. No. 2005/0191698 A1, to each of which further reference should be made.

Sequencing-by-ligation reactions are also useful including, for example, those described in Shendure et al. Science 309:1728-1732 (2005); U.S. Pat. Nos.5,599,675; and 5,750,341, to each of which further reference should be made. Some embodiments can include sequencing-by-hybridization procedures as described, for example, in Bains et al., Journal of Theoretical Biology 135(3), 303-7 (1988); Drmanac et al., Nature Biotechnology 16, 54-58 (1998); Fodor et al., Science 251(4995), 767-773 (1995); and WO 1989/10977, to each of which further reference should be made. In both Sequencing-by-ligation and sequencing-by-hybridization procedures, target nucleic acids (or amplicons thereof) that are present at features of an array are subjected to repeated cycles of oligonucleotide delivery and detection. Fluidic systems for SBS methods as set forth herein or in references cited herein can be readily adapted for delivery of reagents for sequencing-by-ligation or sequencing-by-hybridization procedures. The oligonucleotides may be fluorescently labeled and can be detected using fluorescence detectors similar to those described with regard to SBS procedures herein or in references cited herein.

Some embodiments can utilize methods involving the real-time monitoring of DNA polymerase activity. For example, nucleotide incorporations can be detected through fluorescence resonance energy transfer (FRET) interactions between a fluorophore-bearing polymerase and γ-phosphate-labeled nucleotides. Techniques and reagents for FRET-based sequencing are described, for example, in Levene et al. Science 299, 682-686 (2003); Lundquist et al. Opt. Lett. 33, 1026-1028 (2008); and Korlach et al. Proc. Natl. Acad. Sci. USA 105, 1176-1181 (2008), to which further reference should be made.

The analytes may comprise optically detectable moieties. The plurality of analytes at each of the features may comprise amplicons of a nucleic acid template. Moreover, the analytes may be selected from the group consisting of nucleic acids, proteins, cells, nuclei, carbohydrates, drug candidates and members of a small molecule library.

Regarding the patterns, the first pattern of features may be in a first flat plane and the second pattern of features in a second flat plane that is parallel to the first flat plane. The first pattern of features may be in a first curved plane and the second pattern of features in a second curved plane. Moreover, an area for each of the features of the first pattern may be the same as the area for each of the features of the second pattern. The first pattern has the same pitch as the second pattern. In some embodiments, the patterns may have a configuration selected from the group consisting of a square lattice, rectangular lattice, rhombic lattice, hexagonal lattice and oblique lattice. The features of the first and second patterns may be in simultaneous, fluid communication with a fluid. The features of the first pattern may be optically coupled to a waveguide, while the features of the second pattern are not substantially optically coupled to the waveguide. In such cases, the detecting of the optical signals at the first elevation z1 may comprise optically exciting the analyte features of the first pattern via a waveguide. The analyte features of the second pattern may not be substantially optically coupled to the waveguide.

Further, as noted, the features and support may be provided in or may be part of a flow cell. The exterior surface of the solid support may be in contact with the lumen of the flow cell. The solid support may comprise a first window of the flow cell and the flow cell may comprise a second window that is parallel to the first window, the first window being separated from the second window by the lumen of the flow cell. The second window may comprise a second exterior surface that comprises a third subset of contours positioned along the second exterior surface to form a third pattern of features and a fourth subset of contours along the second exterior surface to form a fourth pattern of features. In such embodiments, the contours of the third subset may be juxtaposed with the contours of the fourth subset along the second exterior surface, whereby the third and fourth patterns form a second interleaved pattern along the second exterior surface, and the features of the third pattern occur at a third elevation z3 and the features of the fourth pattern occur at a fourth elevation z4.

Regarding signals and detection of signals and analytes, the detecting technology may be optical, and may comprise confocal detection. The analyte features in the second pattern may be distinguished from nearest-neighbor analyte features in the first pattern by selectively focusing an optical detector to the second elevation z2 compared to the first elevation z1. Moreover, analyte features in the first pattern may be distinguished from nearest-neighbor analyte features in the second pattern by selectively exciting luminophores at the first elevation z1 compared to the second elevation z2. Analyte features in the second pattern may be distinguished from nearest-neighbor analyte features in the first pattern by selectively exciting luminophores at the second elevation z2 compared to the first elevation z1. Here again, the analyte features may comprise depressions, wells, channels, projections, ridges or posts. The analyte features may also or instead comprise wells and the detecting of the optical signals comprises detecting analytes in the wells. The detecting of the optical signals at the first elevation z1 may comprise detecting analytes in wells having a bottom at elevation z1 and the detecting optical signals at the first elevation z2 may comprise detecting analytes in wells having a bottom at elevation z2. And again, the analyte features may comprise posts and the detecting of the optical signals may comprise detecting analytes on the top of the posts. In such cases, detecting optical signals at the first elevation z1 may comprise detecting analytes on posts having a top at elevation z1 and detecting optical signals at the first elevation z1 may comprise detecting analytes on posts having a top at elevation z2. Detecting of the optical signals may comprise distinguishing features having an area in the xy plane that is smaller than about 1 µm2. The elevations z1 and z2 may be at least about 2 µm apart. The detecting may use a focal plane having a thickness that is less than about 2 µm. In some embodiment, the detecting uses a focal plane having a thickness that is less than the distance between z1 and z2.

Also, for detection, the detecting of signals, such as optical signals, may comprise relative movement between the array and an optical detector in a process of point scanning, line scanning, or step and shoot imaging. The relative movement may be in the xy dimensions. The relative movement for the detecting of the optical signals at the first elevation z1 may be parallel to the relative movement for the detecting of the optical signals at the second elevation z2. Where the exterior surface of the solid support is curved, the relative movement may be along a curved plane. The relative movement for the detecting of the optical signals at the first elevation z1 may be concentric to the relative movement for the detecting of the optical signals at the second elevation z2.

As also discussed above, the technology may also include contacting the array with a fluid comprising a reagent that modifies the analyte features, wherein the features of the first and second patterns are in simultaneous, fluid communication with the fluid. In such cases, the analyte features may comprise nucleic acids and the reagent may comprise a reagent used in a nucleic acid sequencing reaction. The analyte features may comprise nucleic acids and the reagent may comprise complementary nucleic acids that hybridize to the nucleic acids.

The terms "substantially" and "about" used throughout this disclosure, are used to describe and account for small fluctuations, such as due to variations in processing. For example, they can refer to less than or equal to ±5%, such as less than or equal to ±2%, such as less than or equal to ±1%, such as less than or equal to ±0.5%, such as less than or equal to ±0.2%, such as less than or equal to ±0.1%, such as less than or equal to ±0.05%.

The term comprising is intended herein to be open-ended, including not only the recited elements, but further encompassing any additional elements.

A number of embodiments have been described. Nevertheless, it will be understood that various modifications may be made. Accordingly, other embodiments are within the scope of the invention defined by the appended claims.

## Claims

1. An array, comprising:
a solid support;
a first repeating pattern of features positioned along an exterior surface of the solid support and repeating in *x* and *y* dimensions;
a second repeating pattern of features positioned along the exterior surface of the solid support, repeating in the *x* and *y* dimensions, and interleaved with the first pattern of features such that the features of the first repeating pattern are interspersed with features of the second repeating pattern and vice versa, when viewed from a particular perspective;
wherein the first repeating pattern of features comprises posts and the second repeating pattern of features comprises wells;
wherein the posts of the first repeating pattern of features have a top at a first elevation *z1* and the wells of the second repeating pattern of features have a bottom at a second elevation *z2* that is different from *z1* and wherein features present in a particular *z*-plane are separated with spaces between each other,
wherein the posts of the first repeating pattern of features and the wells of the second repeating pattern of features each comprise attachment points for analytes, the posts of the first repeating pattern of features being configured to attach analytes at a different elevation relative to analytes attached to the wells of the second repeating pattern of features,
wherein nearest-neighbor wells of the second repeating pattern of features to each post of the first repeating pattern of features are in an interleaved pattern having a pitch that is less than 500 nm, and
wherein nearest-neighbor posts in the first repeating pattern of features have a pitch that is greater than 500 nm.

2. The array of claim 1, wherein the wells each occupy an area that is smaller than 1 µm².

3. The array of claim 1 or claim 2, wherein *z1* and *z2* are at least 2 µm apart.

4. The array of any preceding claim, wherein the analyte is a nucleic acid analyte and a material that is capable of attaching to the analytes is present at each feature.

5. An array, comprising:
a solid support;
a first repeating pattern of features positioned along an exterior surface of the solid support and repeating in *x* and *y* dimensions;
a second repeating pattern of features positioned along the exterior surface of the solid support, repeating in the *x* and *y* dimensions, and interleaved with the first pattern of features such that the features of the first repeating pattern are interspersed with features of the second repeating pattern and vice versa, when viewed from a particular perspective;
wherein the first repeating pattern of features comprises first posts and the second repeating pattern of features comprises second posts;
wherein the first posts of the first repeating pattern of features have a top at a first elevation *z1* and the second posts of the second repeating pattern of features have a top at a second elevation *z2* that is different from *z1* and wherein features present in a particular *z-*plane are separated with spaces between each other,
wherein the first posts of the first repeating pattern of features and the second posts of the second repeating pattern of features each comprise attachment points for analytes, the first posts of the first repeating pattern of features being configured to attach analytes at a different elevation relative to analytes attached to the second posts of the second repeating pattern of features,
wherein the first posts of the first repeating pattern of features are spaced from the second posts of the second repeating pattern of features;
wherein nearest-neighbor second posts of the second repeating pattern of features to each first post of the first repeating pattern of features are in an interleaved pattern having a pitch that is less than 500 nm, and
wherein nearest-neighbor first posts in the first repeating pattern of features have a pitch that is greater than 500 nm.

6. The array of claim 5, wherein the posts each occupy an area that is smaller than 1 µm².

7. The array of claim 5 or 6, wherein *z1* and *z2* are at least 2 µm apart.

8. The array of any one of claims 5 to 7, wherein the analyte is a nucleic acid analyte and a material that is capable of attaching to the analytes is present at each feature.

## Patentansprüche

1. Ein Array, das Folgendes beinhaltet:
einen festen Träger;
ein erstes sich wiederholendes Muster von Merkmalen, das entlang einer Außenfläche des festen Trägers positioniert ist und sich in den Dimensionen *x* und *y* wiederholt;
ein zweites sich wiederholendes Muster von Merkmalen, das entlang der Außenfläche des festen Trägers positioniert ist, sich in den Dimensionen *x* und *y* wiederholt und mit dem ersten Muster von Merkmalen verschachtelt ist, sodass die Merkmale des ersten sich wiederholenden Musters mit Merkmalen des zweiten sich wiederholenden Musters durchsetzt sind und umgekehrt, wenn sie aus einer bestimmten Perspektive betrachtet werden;
wobei das erste sich wiederholende Muster von Merkmalen Stifte beinhaltet und das zweite sich wiederholende Muster von Merkmalen Vertiefungen beinhaltet;
wobei die Stifte des ersten sich wiederholenden Musters von Merkmalen eine Oberseite auf einer ersten Erhöhung *z1* und die Vertiefungen des zweiten sich wiederholenden Musters von Merkmalen eine Unterseite auf einer zweiten Erhöhung *z2* aufweisen, die sich von *z1* unterscheidet, und wobei Merkmale, die in einer bestimmten Ebene z vorhanden sind, durch Zwischenräume voneinander getrennt sind,
wobei die Stifte des ersten sich wiederholenden Musters von Merkmalen und die Vertiefungen des zweiten sich wiederholenden Musters von Merkmalen jeweils Anheftungspunkte für Analyten beinhalten, wobei die Stifte des ersten sich wiederholenden Musters von Merkmalen so konfiguriert sind, dass sie Analyten auf einer anderen Erhöhung relativ zu Analyten anheften, die an die Vertiefungen des zweiten sich wiederholenden Musters von Merkmalen angeheftet sind,
wobei die zu jedem Stift des ersten sich wiederholenden Musters von Merkmalen am nächsten benachbarten Vertiefungen des zweiten sich wiederholenden Musters von Merkmalen in einem verschachtelten Muster mit einem Abstand, der weniger als 500 nm beträgt, liegen, und
wobei die am nächsten benachbarten Stifte in dem ersten sich wiederholenden Muster von Merkmalen einen Abstand aufweisen, der mehr als 500 nm beträgt.

2. Array gemäß Anspruch 1, wobei die Vertiefungen jeweils einen Flächenbereich einnehmen, der kleiner als 1 µm² ist.

3. Array gemäß Anspruch 1 oder Anspruch 2, wobei *z1* und *z2* mindestens 2 µm voneinander entfernt sind.

4. Array gemäß einem der vorhergehenden Ansprüche, wobei der Analyt ein Nukleinsäure-Analyt ist und ein Material, das sich an die Analyten anheften kann, an jedem Merkmal vorhanden ist.

5. Ein Array, das Folgendes beinhaltet:
einen festen Träger;
ein erstes sich wiederholendes Muster von Merkmalen, das entlang einer Außenfläche des festen Trägers positioniert ist und sich in den Dimensionen *x* und *y* wiederholt;
ein zweites sich wiederholendes Muster von Merkmalen, das entlang der Außenfläche des festen Trägers positioniert ist, sich in den Dimensionen *x* und *y* wiederholt und mit dem ersten Muster von Merkmalen verschachtelt ist, sodass die Merkmale des ersten sich wiederholenden Musters mit Merkmalen des zweiten sich wiederholenden Musters durchsetzt sind und umgekehrt, wenn sie aus einer bestimmten Perspektive betrachtet werden;
wobei das erste sich wiederholende Muster von Merkmalen erste Stifte beinhaltet und das zweite sich wiederholende Muster von Merkmalen zweite Stifte beinhaltet;
wobei die ersten Stifte des ersten sich wiederholenden Musters von Merkmalen eine Oberseite auf einer ersten Erhöhung *z1* und die zweiten Stifte des zweiten sich wiederholenden Musters von Merkmalen eine Oberseite auf einer zweiten Erhöhung *z2* aufweisen, die sich von *z1* unterscheidet, und wobei Merkmale, die in einer bestimmten Ebene *z* vorhanden sind, durch Zwischenräume voneinander getrennt sind,
wobei die ersten Stifte des ersten sich wiederholenden Musters von Merkmalen und die zweiten Stifte des zweiten sich wiederholenden Musters von Merkmalen jeweils Anheftungspunkte für Analyten beinhalten, wobei die ersten Stifte des ersten sich wiederholenden Musters von Merkmalen so konfiguriert sind, dass sie Analyten auf einer anderen Erhöhung relativ zu Analyten anheften, die an die zweiten Stifte des zweiten sich wiederholenden Musters von Merkmalen angeheftet sind,
wobei die ersten Stifte des ersten sich wiederholenden Musters von Merkmalen von den zweiten Stiften des zweiten sich wiederholenden Musters von Merkmalen beabstandet sind;
wobei die zu jedem ersten Stift des ersten sich wiederholenden Musters von Merkmalen am nächsten benachbarten zweiten Stifte des zweiten sich wiederholenden Musters von Merkmalen in einem verschachtelten Muster mit einem Abstand, der weniger als 500 nm beträgt, liegen, und
wobei die am nächsten benachbarten ersten Stifte in dem ersten sich wiederholenden Muster von Merkmalen einen Abstand aufweisen, der mehr als 500 nm beträgt.

6. Array gemäß Anspruch 5, wobei die Stifte jeweils einen Flächenbereich einnehmen, der kleiner als 1 µm² ist.

7. Array gemäß Anspruch 5 oder 6, wobei *z1* und *z2* mindestens 2 µm voneinander entfernt sind.

8. Array gemäß einem der Ansprüche 5 bis 7, wobei der Analyt ein Nukleinsäure-Analyt ist und ein Material, das sich an die Analyten anheften kann, an jedem Merkmal vorhanden ist.

## Revendications

1. Un réseau, comprenant :
un support solide ;
un premier motif se répétant d'éléments positionné le long d'une surface extérieure du support solide et se répétant dans des dimensions *x* et *y* ;
un deuxième motif se répétant d'éléments positionné le long de la surface extérieure du support solide, se répétant dans les dimensions *x* et *y*, et entrelacé avec le premier motif d'éléments de telle sorte que les éléments du premier motif se répétant soient entremêlés d'éléments du deuxième motif se répétant et vice versa, lorsqu'ils sont vus d'un point de vue particulier ;
dans lequel le premier motif se répétant d'éléments comprend des montants et le deuxième motif se répétant d'éléments comprend des puits ;
dans lequel les montants du premier motif se répétant d'éléments ont un dessus à une première élévation *z1* et les puits du deuxième motif se répétant d'éléments ont un fond à une deuxième élévation *z2* qui est différente de *z1* et dans lequel des éléments présents dans un plan *z* particulier sont séparés par des espaces situés entre les uns et les autres,
dans lequel les montants du premier motif se répétant d'éléments et les puits du deuxième motif se répétant d'éléments comprennent chacun des points d'attache pour des analytes, les montants du premier motif se répétant d'éléments étant configurés pour attacher des analytes à une élévation différente par rapport à des analytes attachés aux puits du deuxième motif se répétant d'éléments,
dans lequel des puits voisins les plus proches du deuxième motif se répétant d'éléments par rapport à chaque montant du premier motif se répétant d'éléments sont dans un motif entrelacé ayant un pas qui est inférieur à 500 nm, et
dans lequel des montants voisins les plus proches dans le premier motif se répétant d'éléments ont un pas qui est supérieur à 500 nm.

2. Le réseau de la revendication 1, dans lequel les puits occupent chacun une zone qui est plus petite que 1 µm².

3. Le réseau de la revendication 1 ou de la revendication 2, dans lequel *z1* et *z2* sont séparées l'une de l'autre d'au moins 2 µm.

4. Le réseau de n'importe quelle revendication précédente, dans lequel l'analyte est un analyte acide nucléique et un matériau qui est à même de s'attacher aux analytes est présent au niveau de chaque élément.

5. Un réseau, comprenant :
un support solide ;
un premier motif se répétant d'éléments positionné le long d'une surface extérieure du support solide et se répétant dans des dimensions *x* et *y* ;
un deuxième motif se répétant d'éléments positionné le long de la surface extérieure du support solide, se répétant dans les dimensions *x* et *y*, et entrelacé avec le premier motif d'éléments de telle sorte que les éléments du premier motif se répétant soient entremêlés d'éléments du deuxième motif se répétant et vice versa, lorsqu'ils sont vus d'un point de vue particulier ;
dans lequel le premier motif se répétant d'éléments comprend des premiers montants et le deuxième motif se répétant d'éléments comprend des deuxièmes montants ;
dans lequel les premiers montants du premier motif se répétant d'éléments ont un dessus à une première élévation *z1* et les deuxièmes montants du deuxième motif se répétant d'éléments ont un dessus à une deuxième élévation *z2* qui est différente de *z1* et dans lequel des éléments présents dans un plan z particulier sont séparés par des espaces situés entre les uns et les autres,
dans lequel les premiers montants du premier motif se répétant d'éléments et les deuxièmes montants du deuxième motif se répétant d'éléments comprennent chacun des points d'attache pour des analytes, les premiers montants du premier motif se répétant d'éléments étant configurés pour attacher des analytes à une élévation différente par rapport à des analytes attachés aux deuxièmes montants du deuxième motif se répétant d'éléments,
dans lequel les premiers montants du premier motif se répétant d'éléments sont espacés des deuxièmes montants du deuxième motif se répétant d'éléments ;
dans lequel des deuxièmes montants voisins les plus proches du deuxième motif se répétant d'éléments par rapport à chaque premier montant du premier motif se répétant d'éléments sont dans un motif entrelacé ayant un pas qui est inférieur à 500 nm, et
dans lequel des premiers montants voisins les plus proches dans le premier motif se répétant d'éléments ont un pas qui est supérieur à 500 nm.

6. Le réseau de la revendication 5, dans lequel les montants occupent chacun une zone qui est plus petite que 1 µm².

7. Le réseau de la revendication 5 ou de la revendication 6, dans lequel *z1* et *z2* sont séparées l'une de l'autre d'au moins 2 µm.

8. Le réseau de n'importe laquelle des revendications 5 à 7, dans lequel l'analyte est un analyte acide nucléique et un matériau qui est à même de s'attacher aux analytes est présent au niveau de chaque élément.
